Europäisches Patentamt

European Patent Office

Office européen des brevets

(11) Publication number: **0 001 934**
A2

(12) **EUROPEAN PATENT APPLICATION**

(21) Application number: 78300603.4

(22) Date of filing: 07.11.78

(51) Int. Cl.²: **A 61 B 10/00**
**G 01 S 9/66, G 06 F 15/42**

(30) Priority: 07.11.77 US 848988

(43) Date of publication of application:
16.05.79 Bulletin 79/10

(84) Designated contracting states:
DE FR GB NL

(71) Applicant: LITTON INDUSTRIAL PRODUCTS INC.
360 North Crescent Drive
Beverly Hills California 90210(US)

(72) Inventor: Cribbs, Robert
Lake View Drive
Placerville California(US)

(72) Inventor: Mahony, John
8901A Salmon Falls Drive
Sacramento California(US)

(74) Representative: Godsill, John Kenneth et al,
Haseltine Lake & CO. Hazlitt House 28 Southampton
Buildings Chancery Lane
London WC2A 1AT(GB)

(54) Ultrasonic diagnostic equipment.

(57) Computerized ultrasonic diagnostic equipment is disclosed having operator actuable means (12) for selectively choosing one of a plurality of video processing techniques which are utilized to convert information from ultrasonic pulse reflections into a video displayed image of the interior region of an examined body. To avoid distinct circuits for respective techniques, a digital circuit (100, 200) is provided having an adjustable transfer function to provide certain of the processing techniques.

FIG.1

EP 0001 934 A2

Croydon Printing Company Ltd.

1

# ULTRASONIC DIAGNOSTIC EQUIPMENT

This invention relates to ultrasonic diagnostic equipment particularly suited for medical diagnostic techniques. As is known in the art, ultrasonic systems may be generally described as comprising means for generating a series of ultrasonic pulses into a patient's body, means for detecting reflected pulses, means for deriving and processing information related to the return time and amplitude of each reflection, and means for displaying video images in accordance with the processed information. These systems are capable of displaying images of internal organs of a body which are generally similar in appearance to images derived by x-ray techniques. With the increasing alarm concerning the cumulative effects of exposure to x-rays, ultrasonic techniques have acquired importance as a safe alternative.

The acceptance and use of ultrasonic techniques will largely depend upon the quality of image definition, that is to say, the clarity and accuracy of the imaged information. Image definition is dependent upon the manner by which the reflection signal is processed prior to display. This invention relates, more particularly, to the signal processing of the reflection information prior to its display.

0001934

Many signal processing techniques are known in the art and one or other is used in accordance with the particular body organ under observation, the operating mode of the ultrasonic system, and the type of diagnosis being performed. One possible mode is the A-mode (amplitude versions time). Alternatively, if the location of the edge of an organ, rather than its structure, is of concern the M-mode (motion-mode) may be more suitable in which the motion of a heart valve is to be observed. Yet again, the B-mode (brightness mode) may be applicable when the enlargement of an organ, such as a kidney, is of concern. The B-mode signal-processing technique used will necessarily differ, however, when the structural detail of an organ is the subject of interest. Further, the technique will differ if the organ is the liver, where fine structure is to be viewed, or a fetus where a low dynamic range is needed to eliminate echos generated by the placenta. Additionally, there is a subjective factor whereby different individuals prefer the results of different process techniques.

According to the invention, there is provided an ultrasonic diagnostic system comprising:

ultrasonic pulse generating means for applying a series of pulses to the surface of a body for propogation therethrough;

detecting means (48, 50) for detecting pulse reflections responsive to the emergence of reflected pulses from the body for producing incoming signals indicative of discontinuities in the propagation path; and

video display means responsive to the incoming signals for producing a visual representation of the media defining the propagation path, characterised in that there are:

operator-actuable means (12) for producing a control signal uniquely related to a selected one of a plurality of signal processing methods which condition the incoming signals for video displaying;

0001934

memory means for storing information related to the signal processing methods and responsive to the control signal to read out that portion of the stored information relating to the selected method;

and incoming signal processing means (100, 200, 300, 400, 500) for processing the incoming signal according to said methods, the processing means being operable in accordance with said portion of the stored information to produce for the display means a video signal processed according to the method selected by the operator the processing means including digital processing means (100, 200) having a transfer function which is variable in accordance with data contained in the selected portion of the stored information.

The processing means may comprise means (100) for quantizing the amplitude of incoming signals in accordance with data in said portion of the stored information.

Further, the processing means may also comprise means (200, 500) for receiving the quantized signal and selectively responsive, in accordance with data in said portion of the stored information, to produce as a video signal for the display means either a first signal representative of the quantized signal or a signal representing the subtraction of a second signal from the first, the second signal being a representation of the quantized signal which is time-delayed relative to the first signal.

Thus it is possible to select different methods of processing not merely by selecting different circuits but also by varing selectively the transfer characteristic of one particular circuit, thereby giving the user flexibility without unnecessary duplication of circuitry.

For a better understanding of the invention and to show how the same may be carried into effect, reference will now be made, by way of example, to the accompanying drawings, in which:

Figure 1 is a perspective view of a control console of ultrasonic diagnostic equipment;

Figure 2A and 2B together form a block diagram of a video processing system of the equipment;

Figure 3 is a schematic diagram of a preferred configuration for a digital-to-analog converter of the system of Figures 2A and 2B;

Figure 4 is a schematic diagram of a preferred sample-and-hold circuit arrangement for use with the converter of Figure 3;

Figures 5A and 5B together form a schematic diagram of a digital video processor of the system of Figures 2A and 2B; and

Figure 6 is a schematic diagram of a technique-selecting circuit of the system of Figure 2.

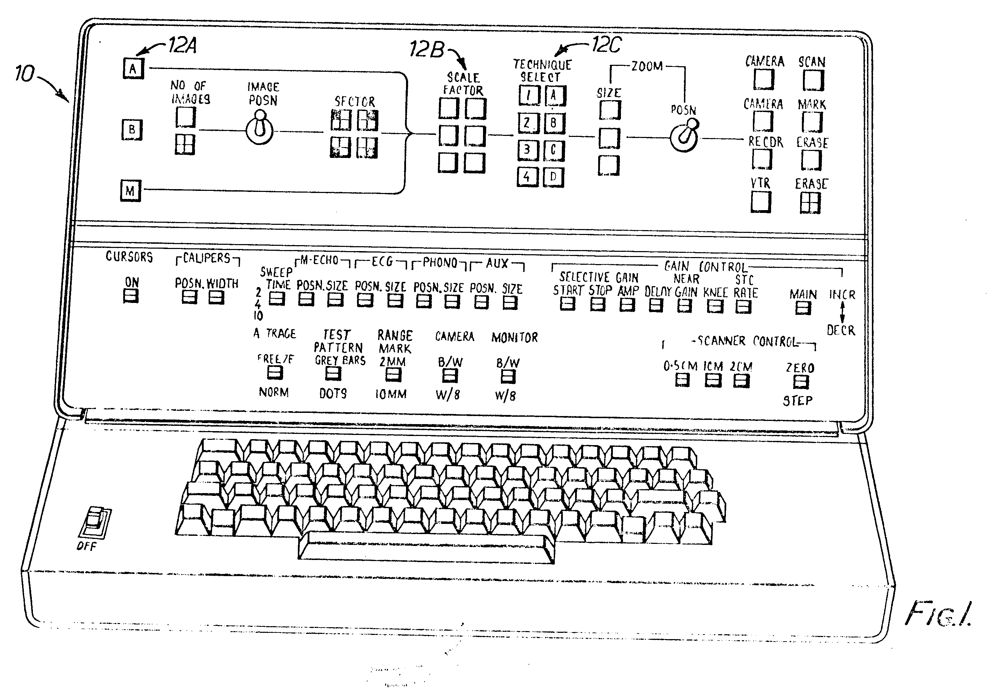

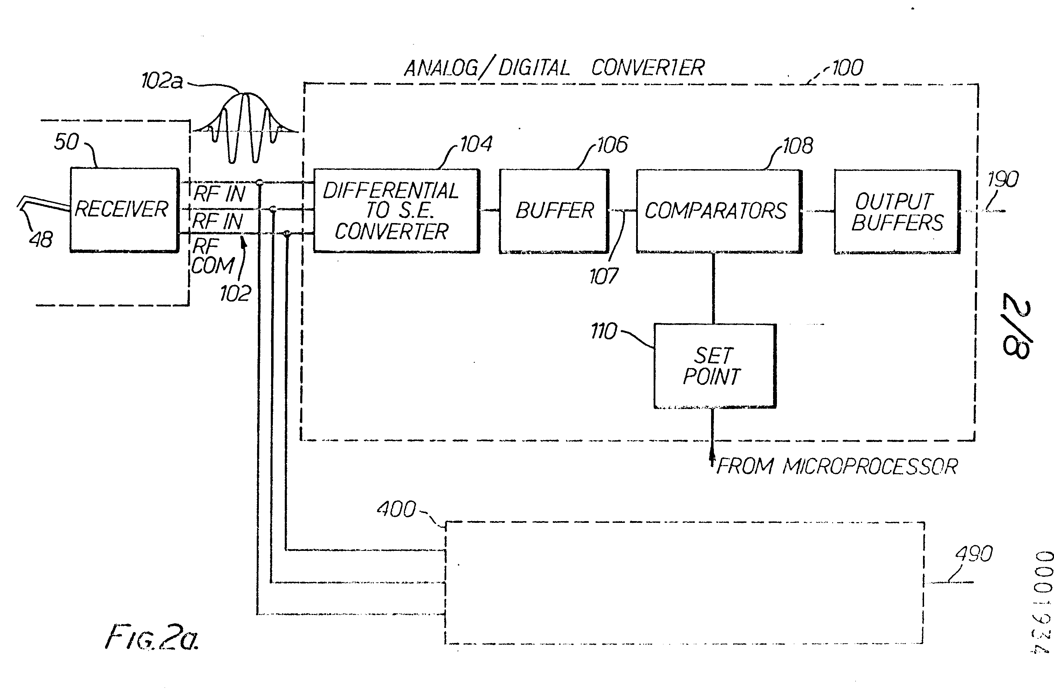

Figure 1 is a perspective view of a control console for use in ultrasonic diagnostic equipment. For the sake of brevity, the functions of the console 10 which are not germane to the present invention are not described. Attention should, however, be directed to push-buttons 12A, labelled A, B and M, scale-factor push buttons 12B and a two-by-four matrix of push-buttons 12C. These buttons provide operator-selectable means by which a desired video processing technique is chosen. Push-buttons 12A enable the operator to select alternative circuitry in the equipment for A-, B- and M-mode processing. The following description relates specifically to the B-mode selection. In the B-mode, various alternatives are available to the operator such as analog and digital techniques and, within digital techniques, alternatives concerning, for example, edge enhancement and dynamic range. For this purpose, the columns of buttons 12C are numerically and alphabetically labelled 1 to 4 and A to D respectively, and it is, of course, obvious that fewer or more buttons may be provided.

For the sake of discussion, it will be assumed that the matrix provides a choice of the following video- processing techniques in the B-mode:

    1.   Analog process

    2.   Digital contour process

    3.   Analog bistable process

    4.   Digital edge-enhancement process

Wherein:

    4A  provides greater dynamic range

    4B  provides small dynamic range

    4C  provides moderate edge-enhancement

    4D  provides greater edge-enhancement

With respect to technique number 4, it is additionally possible to have, for example, combination 4AD which provides increased dynamic range with a large degree of edge enhancement. For each technique selected, a unique control signal (in practice this may be a combination of control signals) is produced which is applied to a microprocessor of the equipment to control the manner by which the information content of the incoming ultrasonic reflections is processed.

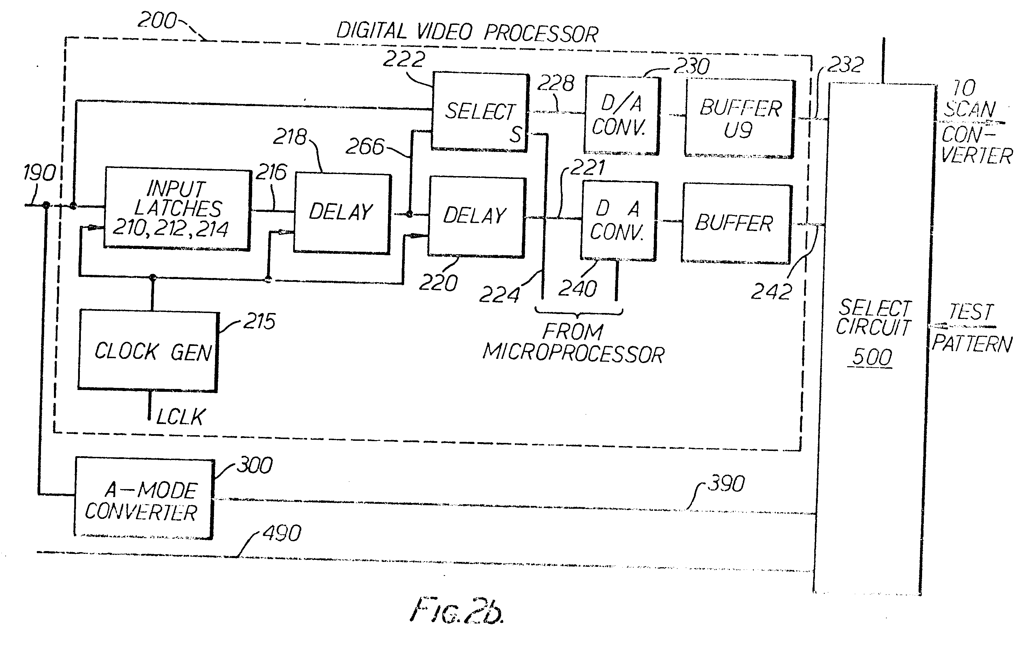

Figures 2A and 2B, together, form a block diagram of the video processing system of the equipment. As will be shown hereinbelow, the microprocessor of the system is responsive to the control signal produced at the console to access a memory associated with the microprocessor and send appropriate instructions and information stored therein to the system hardware. The video processing system is shown to comprise an analog-to-digital converter 100 coupled to an ultrasonic pulse receiver 50 to receive an incoming electrical signal 102 therefrom.

The signal 102 is produced by the receiver 50 in response to ultrasonic reflections, emerging from a body being examined, and detected by a transducer 48. The incoming signal 102 defines a generally pulse shaped envelope 102a, the amplitude characteristics of which are indicative of the degree of change at the reflection-generating discontinuity in the path of the transmitting ultrasonic pulse. The signal is a differential signal on

6

0001934

lines "RF IN" which are at high frequency relative to a common, i.e. ground, potential line "RF COM".

The analog-to-digital converter 100 (the output of which is utilized by select buttons 2 and 4 of the set 12C) is coupled at its output 190 to a digital video processor 200 and to an A-mode converter 300. Also coupled to the receiver 50 is a B and M mode analog processor 400 the output of which is utilized by select buttons 1 and 3 of the set 12C. The processor 200 and 400 and converter 300 are coupled to the input side of a process-selecting circuit 500 which places the appropriate processed video signal at the input of a scan conversion memory for storage and display of the processed information in accordance with any of a variety of methods known in the art.

The converter 100 and processor 200 will now be considered in more detail.

The incoming signal 102 is first quantized by level and converted to a digital form in converter 100. The quantizing levels are determined by pre-programmed values which can be changed as a function of the technique selected e.g. button combinations 4A and 4B. As will be further explained, the output signal from the digital-to-analog converter 100 comprises a plurality of lines, each having a binary value indicative of whether the incoming signal 102 is higher or lower than a value associated with that line, as determined by the pre-programmed values.

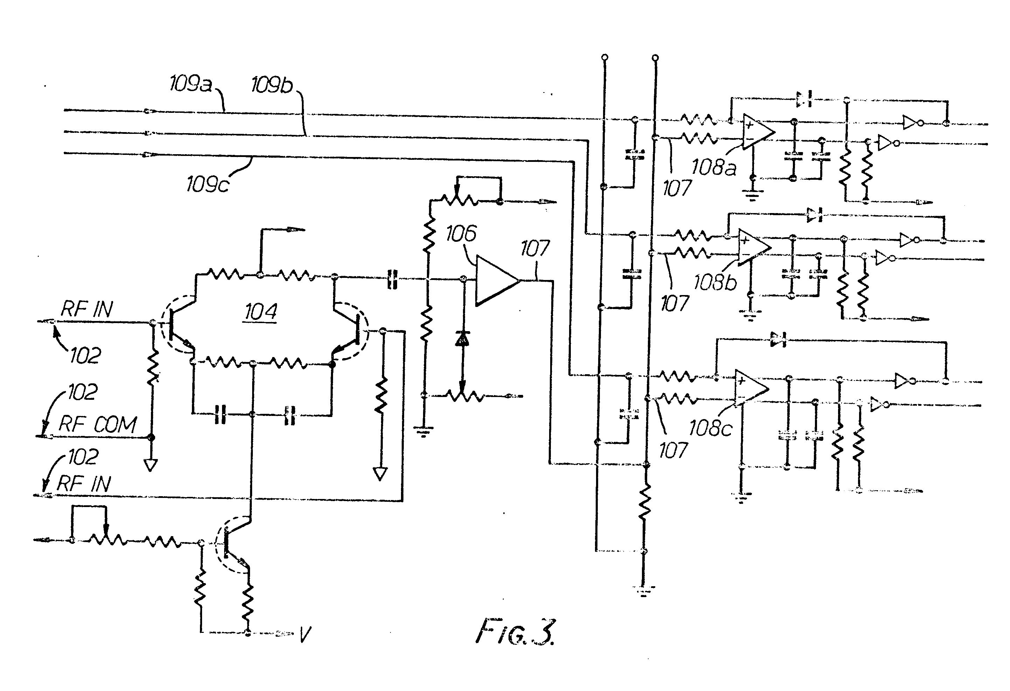

Referring to Figure 3 in conjunction with Figure 2A, the incoming signal 102 is differentially fed by the receiver 50 to a differential-to-single-end converter 104. The converter output 107 passes through a buffer 106 to the inverting input terminals of a plurality of comparators shown in Figure 3 as 108a to 108c. The comparators track the level of the output of the single-ended converter 107 and determine when it is greater than respective set points. Applied to the non-inverting inputs of the comparators 108a to 108c are respective voltage levels 109a to 109c which provide the comparator set points. The values of the plurality of applied voltage levels are pre-programmed into memory at addresses associated with the selected technique, and are part of a greater plurality of stored values which represent all techniques available to the operator for selection.

Since the output of each comparator 108a to 108c is either a "1" or "0", depending on whether the incoming signal is greater than or less than the associated set point, the output from the converter 100 comprises a plurality of binary bits which, together, form a binary word quantizing the instantaneous magnitude of signal 102. In the disclosed embodiment, the comparator outputs assume a "LOW" or "0" binary state when the associated set point value is exceeded. In practice, twelve such comparators have been found to provide optimum results.

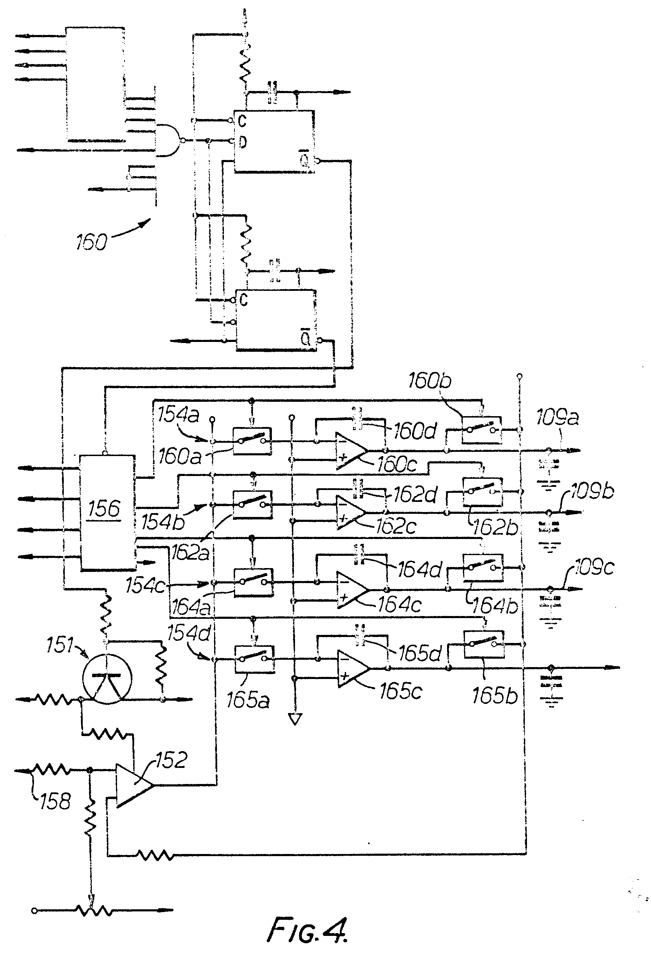

Examining in detail the manner by which the set point voltage levels are applied to the comparators 108a to 108c, reference is made to Figure 4 which depicts, in schematic form, the set point setting circuit 110 as a sample-and-hold circuit. It is demultiplexed-style system wherein a transconductance amplifier 152 drives a plurality of sample-and-hold devices illustratively shown as 154a to 154d. In practice, the number of sample-and-hold devices 154 corresponds to the number of comparators 108 (Figure 3). An analog sample voltage 158 is momentarily applied to the input amplifier 152 which is gated on via switch means 151 in response to gate signals from a timing circuit 160. Gate signals from the timing circuit also control the timing of the opening and closing of individual sample-and-hold switches 160a and 160b, 162a and 162b and 164a and 164b. The appropriate switches are opened and closed by a demultiplexer 156, which is responsive to an address placed at its input.

In operation, the microprocessor accesses the appropriate set point values from memory, in accordance with the selected video processing technique and moment-arily places corresponding analog voltages on the "sample voltage" line 158 while addressing the appropriate sample-and-hold devices 154a to 154d through the demultiplexer 156. The input amplifier 152 is gated "on" to update the addressed sample-and-hold circuit in accordance with input signal 158. After a period of time, the timing circuit 160 shuts the amplifier 152 off, but leaves the demultiplexer 156 on to minimize "glitches" generated by the opening and closing of the sample-and-hold circuit.

In more detail, the sample-and-hold circuits comprise current-responsive digital (CMOS) inverting amp-lifiers 160c to 165c coupled in parallel through switches 160a to 165a to the output of input amplifier 152. Capacitors 160d to 165d are respectively coupled to the inverting amplifiers between the output and inverting input terminals thereof. The output terminals of the inverting amplifiers are respectively coupled through switches 160b to 165b to the non-inverting input of the amplifier 152.

The amplifiers 160c to 165c need not be inverting if the connections to the inverting and non-inverting inputs of amplifier 152 are interchanged and the capacitors 160b to 165b are coupled to common, i.e. ground, potential rather than to the amplifier outputs. The inverting/non-inverting relationship of the amplifiers inputs and outputs are selected so that a change in input signal 158 causes a non-inverted change in the feedback signal.

Referring only to the amplifiers 152 and 160c for the moment, the simultaneous closing of the switches 160a and 160b will cause the output signal 109a to change until it is substantially equivalent to the input signal 158, with offset errors in the amplifier 160c being auto-matically compensated for. Because the gain of the circuit is provided by the input amplifier 152, the amplifier 160c may additionally be one of low gain. Since the gain

0001934

and offset errors of the amplifiers 160c are not critical, the configuration conveniently lends itself to the use of relatively inexpensive CMOS inverters. In the preferred embodiment, RCA 4069's have been chosen as the inverting amplifiers while the switches are provided by CMOS switches (RCA 4016).

In operation, the appropriate portion of the circuit is enabled by a signal from demultiplexer 156 which simultaneously closes the selected switch pair to couple the buffer 152 to a chosen one of amplifiers 160c to 165c.

Means are provided to isolate the held value from the effects of non-simultaneous openings of the sample-and-hold switches.

These means are provided by the timing circuit 160 and demultiplexer 156 to deactivate the input buffer amplifier 152 prior to the opening of the sample-and-hold switches. When the input amplifier 152 is de-energized, no output current flows therefrom and the hold voltage consequently remains fixed. Thus, the opening of the switch 160b before the switch 160a will not affect the held signal value.

The effect of the set point setting circuit 110 is to vary the transfer function of the converter 100 and, therefore, the dynamic range of the system in accordance with the preprogrammed values, in memory, which are associated with each signal-processing technique available for selection.

Returning to Figures 2A and 2B, the output signal 190 from the digital-to-analog converter 100 is a multibit digital word representing the instantaneous magnitude of the incoming signal 102 and with a dynamic range determined by the set points. The output 190 is simultaneously applied to digital video processor 200 and to an A-mode converter 300. The A-mode converter is preferably configured in the manner taught by our co-pending application, filed concurrently herewith, and entitled,

0001934

"Video Display System for Ultrasonic Diagnostic Equipment", the content of which is hereby incorporated by reference. For the purposes of this present description, the A-mode converter 300 may be viewed as a device which converts the unprocessed digital video signal 190 into a signal which is compatible with a video monitor and from which an A-trace may be accordingly displayed upon the selection of the A-mode by the operator.

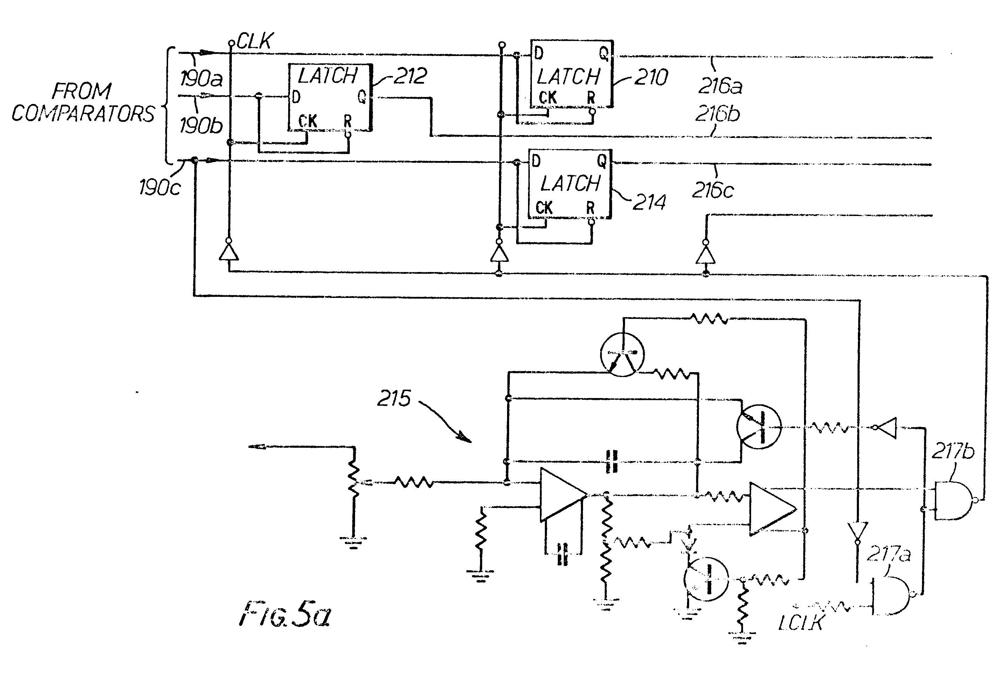

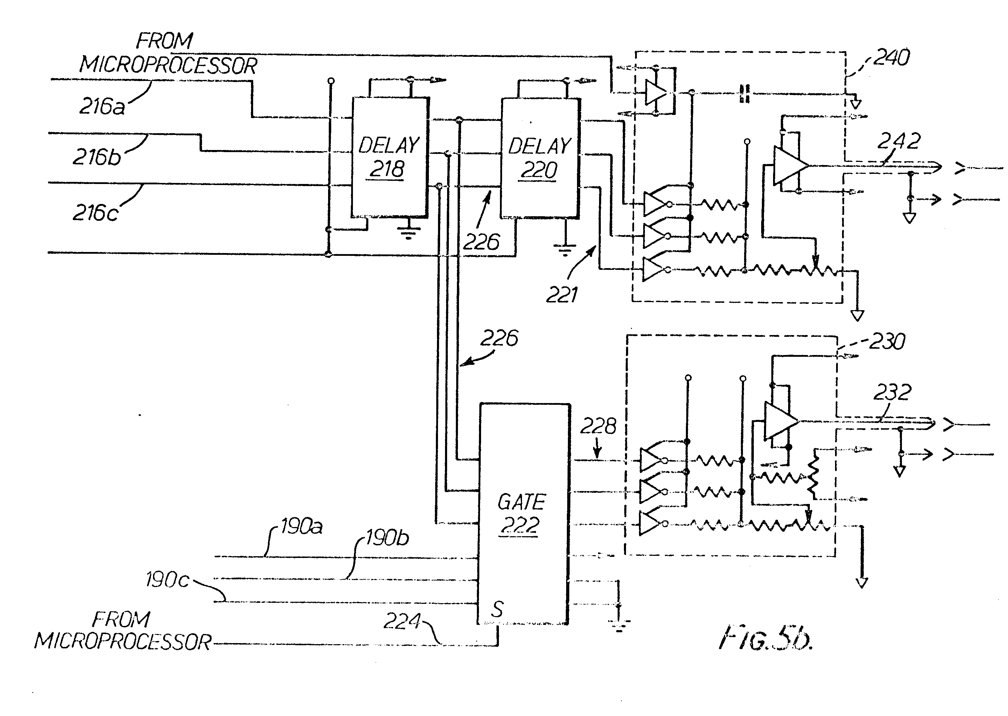

While a general understanding of the digital video processor 200 is obtained by reference to Figures 2A and 2B, a more detailed understanding may be held by reference to Figures 5A and 5B. It is first desirable to detect the peak signal value of each reflection. Naturally, the peak may occur at any time during receipt of the envelope 102a so that the sampling and holding of the incoming signal 102 at a particular discrete time, only, would be likely to miss the peak value. A plurality of latches 210, 212 and 214 are therefore provided to produce an output signal which follows an increasing incoming signal 102, but which clocks down the output signal as the incoming signal 102 decreases. The maximum value is thereby "frozen" for acceptance by remaining circuitry, and, additionally, the latches substantially hold each maximum within the envelope until a larger peak is sampled. The latches are then cleared before the next reflection.

The latches are clocked by a clock generator 215 controlled by a signal LCLK from the microprocessor, LCLK being produced in timed relationship with the ultrasonic pulses emitted by the equipment. Generator 215 is gated by way of gates 217a and 217b when the comparator having the lowest set point is tripped, i.e. comparator 180c in the illustrated embodiment. Each latch 210, 212 and 214 thereafter samples the associated comparator signal 190a to 190c at the rising edge of each clock pulse and, because reset terminal R of each latch is coupled to its input terminal D. is reset when the associated comparator

11

0001934

is tripped by the passing of its set point by the incoming signal 102. The reset latches 210, 212 and 214 remain in their reset state until the incoming signal has decreased below the set point of the associated comparator. The now-activated latches are then clocked to produced the instantaneous signal level on the lines 190a to 190c. Thus, the latches 210, 212 and 214 co-operate to "freeze" the peak value of the incoming signal 102 until the next succeeding circuitry is ready to accept it.

The outputs 216a to 216c from the latches 210, 212 and 214 are passed through one stage of delay 218 and applied in parallel to a second stage of delay 220 and to a selector gate circuit 222. Also applied to the selector circuit 222 are the comparator output signals 190a to 190c. The selector circuit 222 chooses the undelayed signals 190a to 190c when a control signal 224 from the microprocessor indicates that the contour processing has been selected and chooses the once-delayed signal 224 indicates that the image is to be edge-enhanced. In either case, the selected signals 228 are applied to a digital-to-analog converter 230 which produces a video signal 232. In both cases, the video signal 242 from a converter 240 represents the twice-delayed signal 221. The converter 240 is controlled by selected data from the microprocessor to vary the weighting used in its digital-to-analog conversion and also to render its output zero when contour processing is required. The two video signals 232 and 242 are respectively applied as inputs to a technique-select circuit 500.

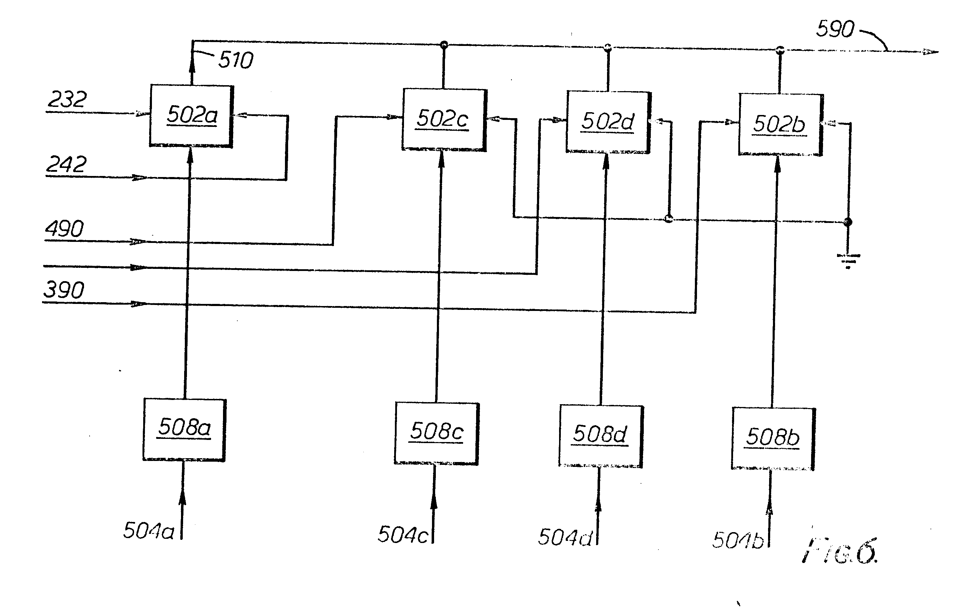

Also applied to the circuit 500 are the output signals from the A-trace converter 300 and the "B and M" Analog Processor 400. Turning to the process selector circuit 500, which applies appropriately processed information to the scan converter for storage and display, attention should be directed to Figure 6. The various video signals 232, 242, 390 and 490 (Figure 2B) are applied as input signals to the selector circuit and are select-

ively gated onto a common output bus 590 in accordance
with the microprocessor memory data corresponding to the
operator-selected mode and technique.

The video signals 232 and 242 from the digital
video processor 200 are applied to opposite inputs of a
differential amplifier 502a. The remaining signals 390
and 490 are applied to respective inputs of respective
differential amplifiers 502b to 502c the opposing inputs
of the latter being coupled together to common potential,
i.e. ground. A further amplifier 502d is provided for
selecting an additional signal for display, for example
a test pattern.

The amplifiers 502a, 502b, 502c and 502d are
selectively activated in accordance with the selected
technique by means of respective selectively-energized
sources 508a to 508d of generally constant current so
that the appropriate video signal is placed on the out-
put bus 590. To energize the proper amplifier, the
system microprocessor addresses a plurality of control
lines 504a to 504d in accordance with the information
accessed from memory in response to the selected tech-
nique and mode. Assuming that the digital technique
with edge enhancement was selected, control line 504a
will go "LOW", while the lines 504b and 504c remain
"HIGH". Transistor 506 is on and amplifier 507 is on,
thereby activating differential amplifier 502a. The
output signal 510 from the amplifier 502a is the difference between video signals 232 and 242. Since the signal
242 is the twice-delayed video signal of selected ampli-
tude, the signal 590 is accordingly the edge-enhanced
signal. If contour processing is selected, amplifier
502a is again selected by   this condition, gate 222
of Figure 5B selects signal 190 and the converter 240
is set to its zero output condition, so that only signal
190 is passed to the bus 590.

While the foregoing description described a pre-
ferred embodiment of the invention, it should be under- stood that many variations and modifications which are obvious to those skilled on the art are included within its scope.

1

0001934

CLAIMS

1.      An ultrasonic diagnostic system comprising:
        ultrasonic pulse generating means for applying
a series of pulses to the surface of a body for propogation therethrough;
        detecting means (48, 50) for detecting reflections
responsive to the emergence of reflected pulses from the
body for producing incoming signals indicative of discontinuities in the propagation path; and
        video display means responsive to the incoming
signals for producing a visual representation of the media
defining the propagation path, characterised in that
there are:
        operator-actuable means (12) for producing a
control signal uniquely related to a selected one of a
plurality of signal processing methods which condition
the incoming signals for video displaying;
        memory means for storing information related
to the signal processing methods and responsive to the
control signal to read out that portion of the stored
information relating to the selected method;
        and incoming signal processing means (100,
2oo, 300, 400, 500) for processing the incoming signal
according to said methods, the processing means being
operable in accordance with said portion of the stored
information to produce for the display means a video
signal processed according to the method selected by
the operator, the processing means including digital
processing means (100, 200) having a transfer function
which is variable in accordance with data contained in the
selected portion of the stored information.

2.      A system according to claim 1, when the processing
means comprises means (100) for quantizing the amplitude
of incoming signals in accordance with data in said
portion of the stored information.

0001934

3.    A system according to claim 2 wherein the in-
coming signal has a multi-peaked waveform defining a
generally pulse-shaped envelope and the quantizing means
includes analog-to-digital conversion means (100) coupled
to the detecting means (48, 50) for producing a digital
output signal (190) representative of the incoming signal
magnitude and means (108) conditioned by said portion of
the stored information for adjusting the dynamic gain of
the conversion means in accordance with said portion.

4.    A system according to claim 3 wherein the conversion means (100) includes: comparators (108) coupled
to the detecting means (48, 50) to receive the incoming
signal and arranged to compare the incoming signal with
respective ones of a plurality of threshhold levels for
producing a binary output signal indicative of whether
the received incoming signal level is higher or lower
than each threshhold level;  and means (110) for applying
the threshhold levels to the comparators, these levels
having been derived from said portion of the stored information.

5.    A system according to claim 4 wherein the applying
means (110) includes sample-and-hold circuitry (154).

6.    A system according to claim 2 or 3, wherein the
quantizing means includes a plurality of comparators
(108) arranged to receive the incoming signal substantially
simultaneously and to compare the incoming signal value
with respective ones of a plurality of threshhold levels,
and a plurality of sample-and-hold circuits (154) arranged
to apply respective threshhold levels to the comparators
and to be intermittently coupled to the memory means for
sampling threshhold level data from said portion of the
stored information.

7.       A system according to any one of claims 2 to 6,
wherein the processing means comprises means (200, 500)
for receiving the quantized signal and selectively
responsive, in accordance with data in said portion of
the stored information, to produce as a video signal for
the display means either a first signal representative
of the quantized signal or a signal representing the
subtraction of a second signal from the first, the second
signal being a representation of the quantized signal
which is time-delayed relative to the first signal.

8.       A system according to claim 7, wherein the
receiving means (200, 500) comprises peak detecting
means (210, 212, 214) for receiving the quantized signal
and for producing a peak signal (216) representing
maxima  of the quantized signal, first and second delay
means (218, 220) for producing from the peak signal first
(226) and second (221) relatively-delayed versions of
the peak signal, selecting means (222) for selecting in
accordance with said portion of the stored information
one or other of the quantized signal and the first delayed version of the peak signal, and means (506),
including subtracting means (502a), operable according
to said portion of the stored information to produce as
said video signal either the output of the selecting
means (222) or the difference between the output of the
selecting means (222) and the second delayed version of
the peak signal (221).

9.       A system according to any one of the preceding
claims, wherein the digital processing means (100, 200)
provides digital B-mode processing and there is additionally
provided an A-mode converter (300), a B-mode analog pro-
cessor (400) and means (500) for selecting between the
digital processing means (100, 200), the A-mode convertor
and the B-mode analog processor in accordance with data
in said portion of the stored information.

12A 12B 12C

10

A
NO OF IMAGES
IMAGE POSN
SECTOR
B
M

SCALE FACTOR

TECHNIQUE SELECT
1 A
2 B
3 C
4 D

SIZE  ZOOM  POSN

CAMERA  SCAN
CAMERA  MARK
RECDR  ERASE
VTR  ERASE

CURSORS  CALIPERS  M-ECHO  ECG  PHONO  AUX  GAIN CONTROL

ON  POSN. WIDTH

SWEEP TIME  POSN. SIZE  POSN. SIZE  POSN. SIZE  POSN. SIZE

SELECTIVE GAIN  NEAR  STC
START STOP AMP DELAY GAIN KNEE RATE  MAIN INCR

2 4 10

DECR

A TRACE  TEST PATTERN  RANGE MARK  CAMERA  MONITOR

FREEZE  GREY BARS  2MM  B/W  B/W

SCANNER CONTROL

0·5CM 1CM 2CM  ZERO

NORM  DOTS  10MM  W/8  W/8  STEP

OFF

Fig.1.

1/8

0001934

ANALOG/DIGITAL CONVERTER ⟋100

RECEIVER 50

48

102a

RF IN
RF IN
RF COM
102

DIFFERENTIAL TO S.E. CONVERTER 104

BUFFER 106

107

COMPARATORS 108

OUTPUT BUFFERS

190

SET POINT 110

FROM MICROPROCESSOR

400

490

FIG.2a.

Fig.2b.

DIGITAL VIDEO PROCESSOR

INPUT LATCHES 210,212,214

DELAY

SELECT S

D/A CONV.

BUFFER U9

DELAY

D A CONV.

BUFFER

CLOCK GEN

LCLK

A—MODE CONVERTER

SELECT CIRCUIT 500

TO SCAN CON-VERTER

TEST PATTERN

FROM MICROPROCESSOR

FIG.3.

5/8

0001934

FIG.4.

FROM COMPARATORS

Fig.5a

8/9

Fig.5b.

Fig.6.